# EUROPEAN PATENT APPLICATION

(11) **EP 4 691 448 A1**
(43) Date of publication of application: **11.02.2026**
(21) Application number: 24382880.3
(22) Date of filing: 05.08.2024
(51) Int. Cl.: A61K 6/17, A61K 6/50, A61K 6/52, A61K 6/54, A61K 6/76, C01B 33/141

(54) **SUBSTANCE FOR THE TREATMENT OF AN ENDODONTIC INFECTION**

(71) Applicant: Universitat Internacional De Catalunya, Fundació Privada, 08017 Barcelona (ES)
(72) Inventor: DELGADO GAROÑA, Luis Maria, 08017 BARCELONA (ES); PÉREZ ANTOÑANZAS, Roman, 080117 BARCELONA (ES); DEMIQUELS PUNZANO, Elena, 08017 BARCELONA (ES); GONZÁLEZ SÁNCHEZ, José Antonio, 08017 BARCELONA (ES); DURÁN-SINDREU TEROL, Fernando, 08017 Barcelona (ES)
(74) Representative: Torner, Juncosa I Associats, SL

(57) **Abstract**

The present disclosure relates to a substance for use in the treatment of an endodontic infection, wherein the substance comprises submicron silica particles. The present disclosure also relates to a method to obtain such substance, the method comprising the following steps: mixing an alcohol with ammonium hydroxide, adding a silicon alkoxide to the mixture, stirring the resulting mixture to obtain a solid and a liquid phase, separating the solid phase by centrifugation and removing the resulting supernatant, redispersing the solid phase in water, and sonicating the re-dispersed solid phase.

## Description

### TECHNICAL FIELD

The present disclosure relates to a substance for the treatment of an endodontic infection and to a method to obtain the substance thereof.

### BACKGROUND ART

The root canal is a complex system, which does not contain commensal microbiota, as in the case of the oral cavity, so any microbial invasion can trigger pathological outcomes. Microorganisms, especially bacteria, can commonly reach dental pulp through dentinal tubules, which are microscopic channels from 0.6 to 2.0 µm in diameter, and bacteria can stay in strategic positions causing resistant endodontic infections. When these microbial communities reach to peri-radicular tissues, can initiate inflammatory responses, causing tissue lysis, pain and necrosis.

Endodontic treatment, also known as root canal treatment, refers to any procedure used to restore and treat teeth where pulp has been injured or exposed, as well as the effective elimination of bacteria in the root canal system and the prevention of their regrowth. An effective cleaning and shaping process of root canals are essential and it can be normally done through various mechanical instrumentation and irrigation techniques. However, the use of these techniques can become a great challenge to completely eradicate any bacteria due to the complexity of root canals anatomy, even when chemical disinfectant irrigants are used. Moreover, the most common chemical irrigants present some limitations; chlorhexidine is associated with reversible discoloration, cannot dissolve organic debris and it can be associated with necrosis at larger doses, while sodium hypochlorite can damage vital tissues and periodontal ligament damage at high concentrations.

Complementary to the mechanical instrumentation and irrigation techniques, calcium hydroxide is widely used as an intracanal medicament to completely disinfect the area and seal root canal to prevent reinfections between medical appointments. Nonetheless, *Enterococcus faecalis* (*E. faecalis*) is less susceptible to calcium hydroxide than other bacteria, despite extensive treatment. Another successful root canal treatment, consists in a combination of antibiotics (ciprofloxacin, metronidazole and minocycline), called 'triple antibiotic paste' (TAP), capable to eradicate anaerobe and aerobe bacteria. However, TAP presents some disadvantages as resistant bacterial development and tooth discoloration due to the presence of minocycline.

Despite endodontic treatment has a high success rate, treatment failure does occur in a significant number of cases due to an inadequate mechanical debridement and specially to bacterial persistence in dentine canals. This study is focused on secondary or persistent infections that can lead in the failure of endodontic treatments. One of the main bacteria associated with these types of infections is *E. faecalis* strain, which is an anaerobic gram-positive coccus that may cause severe tissue damage leading to endodontic treatment failure, showing the capacity of surviving and colonizing the root canal system.

Nowadays in dentistry, extensive research has been directed towards the application of micro and nanotechnology, presenting a myriad of advantages. This innovative approach in dentistry holds promise due to its physiological and pharmacokinetic benefits, aiming to enhance efficacy, tolerability, and patient compliance. Addressing the formidable challenges in achieving complete biofilm elimination, tissue regeneration, and restoration of native tissue sealing, particularly in the realm of endodontics, has become imperative.

Recent studies have proposed new antibacterial strategies, developing root canal resin-based sealants combined with calcium phosphate to promote mineralization, achieving a comprehensive bacterial biofilm elimination through mechanical and chemical instrumentation; or innovative intracanal medications, such as poly(lactic-co-glycolic acid) nanoparticles with encapsulated calcium hydroxide. Furthermore, recent advances have been focused on dual therapeutic strategies developing calcium-silicate nanoparticles with antibacterial and remineralization properties as a potential application of filling an apical root canal of a tooth. However, it has been demonstrated that calcium silicate-based materials can present some drawbacks such the risk of teeth discoloration.

Mesoporous silica nanoparticles are highly suitable for drug delivery due to their uniform porous structure that allows precise control over drug loading and release kinetics, a large pore volume capable of accommodating pharmaceuticals, a high surface area facilitating drug adsorption, and a surface rich in silanol groups that can be modified to optimize drug loading and release.

For instance, US2018050115 A1 discloses silica nanoparticles in the form of multi-layered silica shells encapsulating one or more bioactive ingredients.

EP304643 A1 discloses toroidal mesoporous silica nanoparticles comprising a central cavity of about 10 nm to about 200 nm, the nanoparticles having a size of about 25 nm to about 300 nm.

However, there is still need in the art silica particles that have antibacterial properties and might actively stimulate hard tissues regeneration, positioning them as promising candidates for the development of advanced biomaterials to enhance tissue regeneration after bacterial infection.

### SUMMARY OF THE DISCLOSURE

The present disclosure relates, in a first aspect, to a substance for use in the treatment of an endodontic infection, wherein the substance comprises submicron silica particles.

The present disclosure relates, in a second aspect, to a method to obtain the substance disclosed herein, the method comprising the following steps: a) Mixing an alcohol with ammonium hydroxide, b) Adding a silicon alkoxide to the mixture, c) Stirring the resulting mixture to obtain a solid and a liquid phase, d) Separating the solid phase by centrifugation and removing of the resulting supernatant, e) Redispersing the solid phase in water, and f) sonicating the re-dispersed solid phase.

### BRIEF DESCRIPTION OF THE FIGURES

The foregoing and other advantages and features will be more fully understood from the following detailed description of an embodiment with reference to the accompanying drawings, to be taken in an illustrative and non-limitative manner, in which:

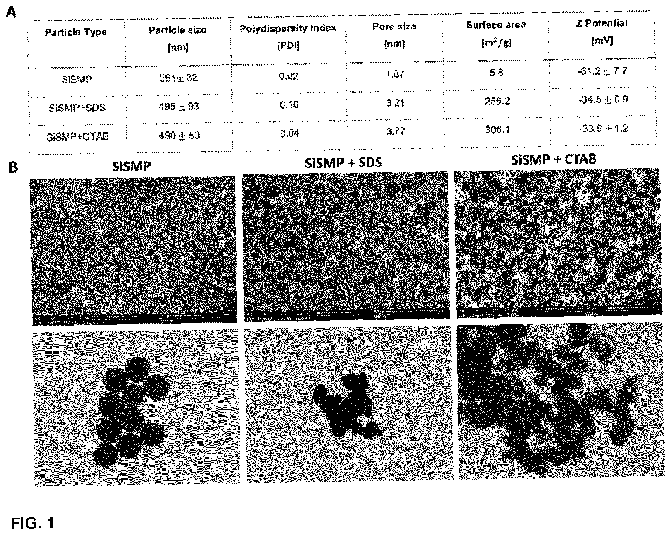

FIG.1 illustrates the characterization of silica submicron particles synthesized using SDS and CTAB, in which FIG. 1A discloses determination of particle size, polydispersity, pore size, surface area and charge while FIG. 1B shows SEM and TEM analysis. For SEM images, scale bars indicate 50 µm. For TEM images, scale bars indicate 1 µm, 2 µm and 500 nm, respectively.

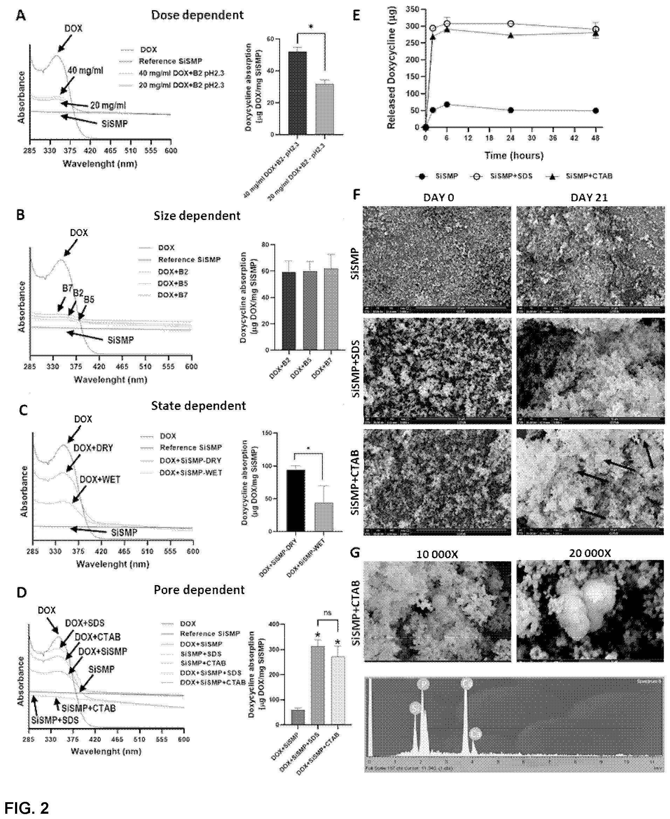

FIG. 2 illustrates doxycycline loading into and release from SiSMP and characterisation of SiSMP biomineralisation capacity. Doxycycline loading into SiSMP is depicted as a function of doxycycline concentration (FIG. 2A), particle size (FIG. 2B), particle state (FIG. 2C) and pore type (FIG. 2D); left graph represents the absorbance spectrum of the particles, and the right graph represents the quantification of the doxycycline loading capacity. FIG. 2E shows the accumulative doxycycline release from the different SiSMPs. SEM images of the biomineralisation assay at day 0 and 21 of the different SiSMPs are shown in FIG. 2F, detailed images of the precipitates for SiSMP+CTAB and EDX analysis of these precipitates are shown in FIG. 2G.

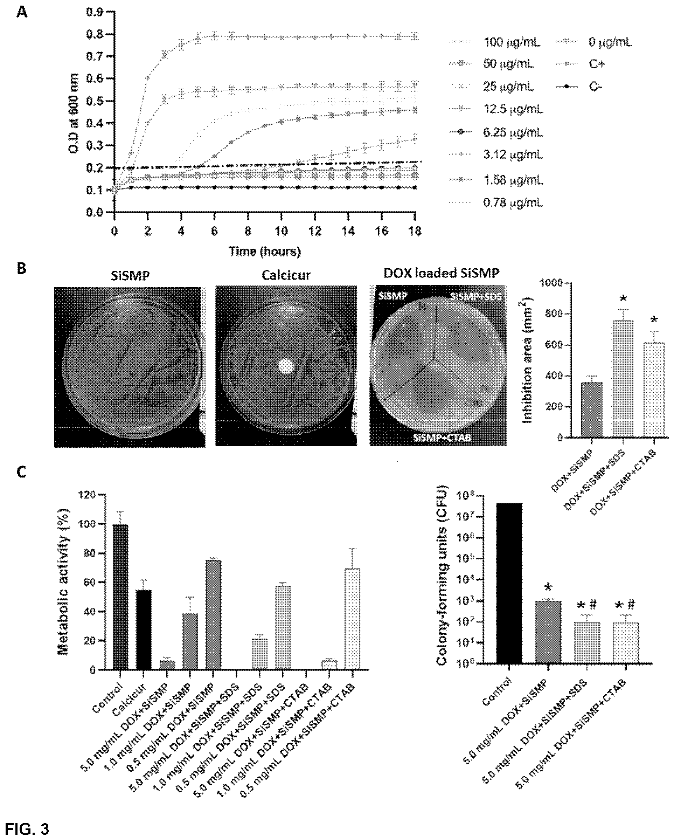

FIG. 3 illustrates the *in vitro* antibacterial activity evaluation of the different DOX loaded SiSMPs. The minimal inhibitory concentration of doxycycline for *E. faecalis* is shown in FIG. 3A. Agar diffusion test and quantification of the inhibition area for the different DOX loaded SiSMPs, using SiSMPs and a commercially available intracanal medication (Calcicur) as control is shown in FIG. 3B. * indicates statistically significant difference compared to DOX+SiSMP (p<0.05). Assessment of the antibacterial activity in 2D in vitro culture measured by metabolic activity and quantification of the colony forming units (CFU) is shown in FIG. 3C. * and # indicate statistically significant difference compared to Control and DOX+SiSMP (p<0.05), respectively.

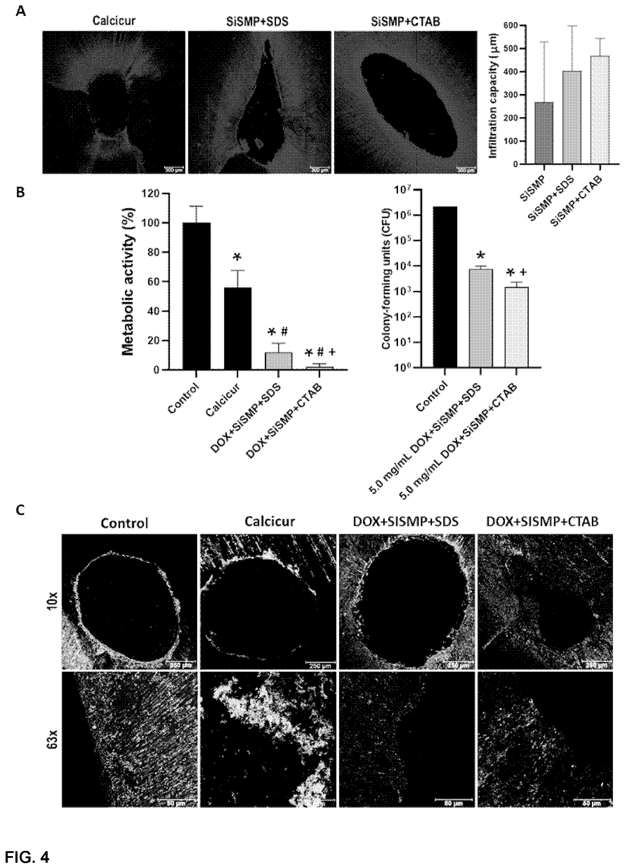

FIG. 4 illustrates ex *vivo* antibacterial activity assessment of the different SiSMPs. Infiltration capacity of SiSMP+SDS and SiSMP+CTAB is shown in FIG. 4A, Calcicur was used a commercial control. Metabolic activity and colony forming units (CFU) of teeth infected with *E. faecalis* for 21 days and treated with SiSMP+SDS, SiSMP+CTAB and Calcicur is shown in
FIG. 4B. Confocal images of Live/Dead staining apical section infected with *E. faecalis* for 21 days and treated with SiSMP+SDS, SiSMP+CTAB and Calcicur is shown in FIG. 4C. Bright light grey account for alive bacteria, while dull dark grey account for dead bacteria. *, # and + indicate statistically significant difference compared to Control, DOX+SiSMP and DOX+SiSMP+SDS (p<0.05), respectively.

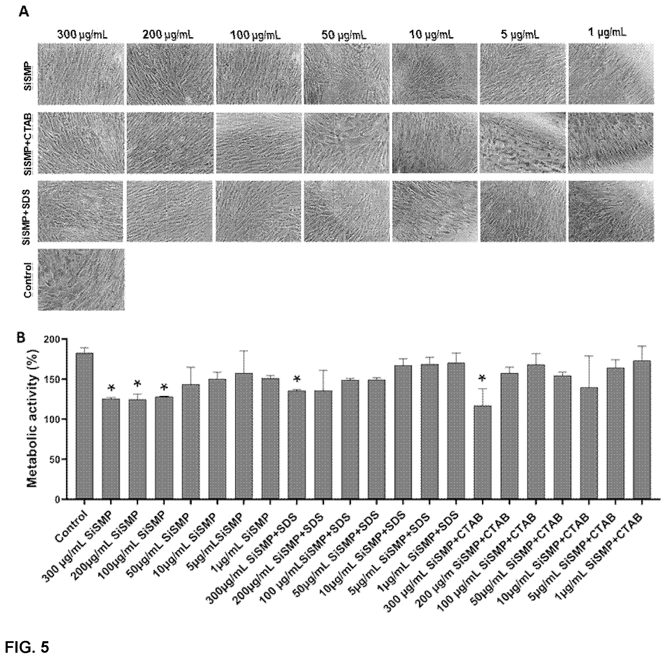

FIG. 5 illustrates human fibroblast (HFF-1) cells seeded with the different silica submicron particle types and at different concentrations (1-300 µg/mL) at day 7. Cell morphology is shown in FIG. 5A, and metabolic activity is shown in FIG. 5B.

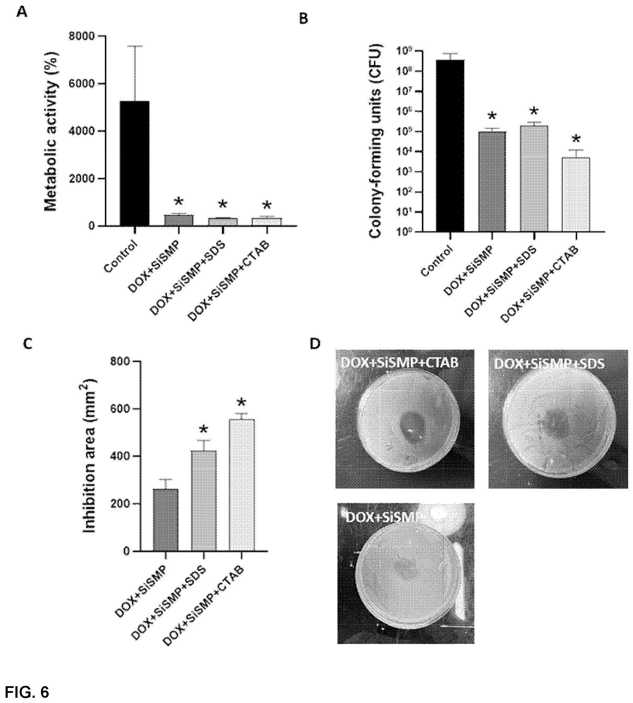

FIG. 6 illustrates *in vitro* antibacterial activity evaluation of the different DOX loaded SiSMPs against *P*. *gingivalis.* Assessment of the antibacterial activity in 2D in vitro culture measured by metabolic activity is shown in FIG. 6A and quantification of the colony forming units (CFU) is shown in FIG. 6B. Agar diffusion test and quantification of the inhibition area for the different DOX loaded SiSMPs are shown in FIGs. 6C and 6D. * indicates statistically significant difference compared to DOX+SiSMP (p<0.05).

### DETAILED DESCRIPTION OF THE DISCLOSURE

The present disclosure relates to a substance for use in the treatment of an endodontic infection, wherein the substance comprises submicron silica particles.

Therefore, the present disclosure relates to a second medical use of a known substance, i.e., submicron silica particles.

Endodontic infections are the infection of the dental root canal system and the major etiologic agent of apical periodontitis. Endodontic infections are polymicrobial and are made up of predominantly anaerobic bacteria and some facultative bacteria. A tooth with an infected nonvital pulp is a reservoir of infection that is isolated from the patient's immune response and will eventually produce a periradicular inflammatory response.

Submicron silica particles are generally defined as silica particles having a particle size distribution less than 1 micron. They are differentiated from silica nanoparticles in that their size is substantially higher - silica nanoparticles are generally understood as particles having a particle size of about 150 nm or less.

In particular, the submicron silica particles for use in the treatment of an endodontic infection are spherical and/or polygonal and/or have a distribution size of between 400 and 1200 nm, preferably between 400 and 800 nm, even more preferably between 400 and 600 nm.

The submicron silica particles as defined herein may incorporate a surfactant, preferably selected from the group of cationic surfactants, anionics surfactants and/or combinations thereof.

A preferred concentration of surfactant may be in the range of between 0.05 to 0. 5 M for both surfactants, preferably 0.14 M for SDS and 0.22 M for CTAB.

A preferred cationic surfactant according to the present disclosure may be cetyl trimethyl ammonium bromide (CTAB) while a preferred anionic surfactant according to the present disclosure may be sodium dodecyl sulfate (SDS).

Cetyl trimethyl ammonium bromide is also known in the art as Cetrimonium bromide.

A particular therapeutic use have been surprisingly found when the submicron silica particles incorporate CTAB therein, which is the ability to regenerate dental bone tissue.

Therefore, the present disclosure relates to a substance for use in the treatment of an endodontic infection and for use in the regeneration of dental bone tissue, wherein the substance comprises submicron silica particles that incorporate the surfactant cetyl trimethyl ammonium bromide.

Moreover, the regeneration of dental bone tissue may preferably involve the formation of calcium apatite on the tissue. Calcium apatite is deposited by precipitation when the infected dental bone tissue is treated with submicron silica particles incorporating CTAB.

The substance according to the disclosure may further encapsulate an antibiotic. The antibiotic may be selected from a wide variety of antibiotics available to the skilled in the art. A preferred antibiotic is doxycycline.

The endodontic infection as disclosed herein may be caused by a wide variety of agents, and in particular, the endodontic infection may be caused by *Enterococcus faecalis (E. faecalis)*, *Porphyromonas Gingivalis (P Gingivalis), Fusobacterium Nucleatum (F. Nucleatum), Pseudomonas aeruginosa (P. aeruginosa),* other *Streptococci* and/or *Prevotella* species and/or combinations thereof.

In particular, the endodontic infection that is treated by the submicron silica particles as disclosed herein is a secondary or persistent endodontic infection. Secondary endodontic infections are caused by microorganisms introduced into the root canal secondarily to professional intervention, either iatrogenically during operative procedures, or later by coronal percolation via micro-leaking restorations, as opposed to primary endodontic infections, typically caused by microorganisms that initially invade and colonize the necrotic pulp, characterized by a polymicrobial infection dominated by anaerobic bacteria.

In particular, the endodontic infection may be located in the dentinal tubule.

The present disclosure also relates to a method to obtain the substance for use in the treatment of an endodontic infection. In particular, the method, derived from the Ströber method, may comprise the following steps: a) Mixing an alcohol with ammonium hydroxide, b) adding a silicon alkoxide to the mixture, c) Stirring the resulting mixture to obtain a solid and a liquid phase, d) Separating the solid phase by centrifugation and removing of the resulting supernatant, e) Redispersing the solid phase in water, and f) sonicating the re-dispersed solid phase.

The alcohol may preferably be pure ethanol; the silicon alkoxide is preferably tetraethyl orthosilicate.

In a preferred embodiment, steps b) and c) take place at about 30 °C. Preferably, the process of obtaining the submicron silica particles herein disclosed is not performed at higher temperatures than 40°C.

The method as disclosed herein may further comprise a step of adding a solution in alcohol of a surfactant prior to step c), so that submicron silica particles incorporating a surfactant may be obtained. The alcohol may be pure ethanol.

The method may also comprise a step of adding hydrochloric acid after step f).

Moreover, the method may comprise a step of adding an antibiotic solution prior to step c), so that submicron silica particles encapsulating an antibiotic may be obtained.

The method as disclosed herein may involve various subsequent repetitions of steps d), e) and f), so that the solid phase, that contains the submicron silica particles is clean and pure. Those iterations might be at least two times, but repetitions even up to seven are plausible.

As shown below in the Examples section, the submicron silica particles disclosed herein show promising antibacterial activity and, when CTAB is the surfactant incorporated therein, bone regeneration takes place. These particles have better performance than Calcicur, a substance based on calcium hydroxide that is currently the gold standard against endodontic infections.

Therefore, the substance as disclosed herein has antimicrobial applications but also is able to induce biomineralization in specific embodiments, that is, when CTAB is present. The biomineralization process can be performed by the silica particles without the use of calcium to contribute to the reparative aspects of damaged teeth, providing therefore with a treatment with dual therapeutic capacity. This dual-purpose approach aligns with the broader scope of advanced therapeutic modalities in regenerative medicine.

### EXAMPLES

### Example 1: synthesis of silica submicron particles (SiSMP)

Silica submicron particles were synthetized adapting the Ströber procedure as disclosed herein. For the preparation, 41.5 mL of ethanol absolute pure and 12.2 mL of ammonium hydroxide (NH₄OH, 28%) were mixed at 30°C in a glass flask. Then, 2.1 mL of tetraethyl orthosilicate (TEOS) was added into the solution and kept under stirring at 375 rpm and at 30°C for 60 min. Then, solution was transferred into two different falcons and the solid phase was separated by centrifugation at 4 000 rpm for 5 min. After removal of the supernatant, the solid was re-dispersed in 25 mL of fresh milliQ water and both falcon tubes were vortex and added into the sonication bath. Centrifugation, re-dispersion, and sonication was repeated six times with milliQ water and three with ethanol absolute pure. The silica particles synthetized under these conditions were named B2.

For a better procedure understanding, it was decided to repeat the synthetization changing the temperature, TEOS quantity and speed parameters (B1, B3, B4, B5, B6 and B7) seen in Table 1. Furthermore, in all the different procedures, the reaction was also stopped for 20 and 40 min. In each of these time points 1 mL was transferred in an Eppendorf tube and then, the same steps as for the 60 min were performed.

**Table 1. Optimization of synthesis parameters to control particle size of silica submicron particles.**

| **Batch number** | **Temperature [°C]** | **Speed [rpm]** | **TEOS [mL]** |
|---|---|---|---|
| B1 | 20 | 375 | 2.1 |
| B2 | 30 | 375 | 2.1 |
| B3 | 40 | 375 | 2.1 |
| B4 | 50 | 375 | 2.1 |
| B5 | 30 | 500 | 2.1 |
| B6 | 30 | 250 | 2.1 |
| B7 | 30 | 250 | 4.2 |

All the formulated silica particles exhibited a round and regular shape. In addition, each particle batch showed regular distribution sizes between 450 and 1200 nm.

Regarding the synthesis conditions used, it was demonstrated that as agitation speed increased, particle size decreased and vice versa. In addition, it was observed that the silica precursor concentration increased particle size. Nonetheless, temperature and synthesis time did not seem to significantly influence either size or morphology of particles. For this reason, these results suggest particle synthesis time could be reduced. According to the results obtained in previous studies, both re-dispersion in ethanol and washing with water steps have a significant impact on pore formation, since silica rearranges throughout that stage, and treatment in alcohol seems to be crucial. Moreover, all studied groups showed very similar characteristics to those obtained in the afore-mentioned study, with synthesis parameters similar to B2 through the Ströber method. Extremely narrow particle size distributions were demonstrated, and average particle diameter was around 561 nm.

For drug encapsulation studies, three batches were selected for further analysis: B2, as intermediate particle size and synthesized under well established; B5 as the smallest particle size, and B7 as the largest one.

### Example 2: synthesis of silica submicron particles using SDS and CTAB

In the pursuit of fine-tuning particle structure, silica particles were successfully synthesized using two distinct surfactants. The incorporation of cationic surfactant, CTAB, and an anionic surfactant, SDS, during the synthesis process allowed particles modification, acting as pore template phase. The unmodified silica particles, previously referred as the B2 group, were renamed SiSMP; while SDS and CTAB modified particles were named as SiSMP+SDS and SiSMP+CTAB, respectively.

Silica submicron particles with surfactants CTAB and SDS were synthetized under the same conditions as B2, previously described. For this preparation, 0.4 g of sodium dodecyl sulphate (SDS) and 0.8 g of cetyltrimethylammonium bromide (CTAB, 99%) were dissolved separately in 10 mL of ethanol absolute pure in a glass flask. Then, 31.5 mL of ethanol absolute pure and 12.2 mL of NH₄OH,28% were mixed at 30°C in a glass flask. Then, 2.1 mL of TEOS was added into the solutions, and left to react under stirring for 60 min. Then, the prepared solutions were transferred into different falcons and the solid phase was separated by centrifugation at 4 000 rpm for 5 min. After removal of the supernatant, the solid was re-dispersed in 25 mL of fresh milliQ water and falcon tubes were vortex and added into the sonication bath. Centrifugation, re-dispersion and sonication were repeated nine times in milliQ water and six times with ethanol absolute pure. To completely assure the surfactants removal, 1 M hydrochloric acid was added to each solution after the seventh wash and left stirring overnight at 50°C.

The pore size modification was confirmed by the N₂ adsorption-desorption isotherms analysis where SiSMP+SDS and SiSMP+CTAB particles significantly had larger pore size and surface areas than SiSMP, as can be seen in FIG. 1A. Moreover, the synthesis using surfactant significantly reduced surface charge for both SiSMP synthesized with surfactants (p<0.05). Regarding the particle size, surfactants also induced some alterations, SDS increased size heterogeneity as observed in the polydispersity index and TEM images, while CTAB slightly reduced particle size (p<0.05).

FIG. 1B presents SEM and TEM images of the different particles synthetized with and without surfactants. Both SiSMP and SiSMP+SDS exhibited a round shape with a smooth surface. In contrast, SiSMP+CTAB exhibited a more irregular and polygonal shape. Additionally, both SiSMP+SDS and SiSMP+CTAB showed a clear tendency to agglomerate, likely due to the reduction of surface charge. Although it was not possible to definitely determine the pore morphology of the particles synthesized with SDS and CTAB under present TEM conditions, previous studies reported different particle and pore morphologies when these surfactants were introducing during synthesis. Specifically, SDS can induce a star-like shape with non-uniform sizes and tendency to agglomerate, consistent with the present disclosure. On the other hand, CTAB modified particles can induce hexagonal pore structures, with the CTAB concentration needing careful adjustment to avoid the formation of silica nanorods. EDX analysis did not reveal traces of sodium or bromide from the surfactant, confirming that washing steps and the acid treatment completely removed the surfactant template.

Scanning electron microscopy (SEM, FEI Quanta 200) and transmission electronic microscopy (TEM, JEOL JEM 1010 at 100 KV) was used to examine the particles morphology. The particle size, polydispersity index and zeta potential of each type of particle was determined by Dynamic Light Scattering (DLS, Malvern Zetasizer). Also, N₂ adsorption-desorption isotherms analysis was performed to determine pore sizes and specific surface areas.

### Example 3: Doxycycline loading into SiSMP

Two different DOX concentrations were prepared (20 and 40 mg/mL) and different pH values (2.3 and 5.5) were investigated. For the encapsulation, 500 µl of each type of DOX and 100 µl of SiSMP were added in different Eppendorf tubes. The mixture was left under stirring at 4 °C for 1 day. Then, centrifugation was performed 10 min at 10000 rpm for the obtention of DOX-loaded SiSMP. To calculate drug loading and its efficiency, each supernatant of the experimental groups (i.e., 40 mg/mL-DOX-ph2.3, 20 mg/mL-DOX-ph2.3...) and of the control ones (i.e., DOX alone) were transferred into a 96-well plate UV and measured at 350 nm wavelength and full scanned from 285 to 600 nm wavelength at the spectrophotometer (Infinite M nano +, TECAN). The amount of loaded DOX was calculated using a standard curve and comparing the DOX concentration before and after the particle incubation. Values were normalized to the SiSMP weight in mg. This procedure was repeated to assess the effect of particle size, state and pore modification.

To determine the encapsulation efficiency of the different silica particles, series of optimization (drug concentration, particle synthesis, state and mesoporosity) were performed using doxycycline (DOX) as model drug and due to its efficiency against *E. faecalis* and *P. gingivalis.*

First, the encapsulation efficiency of silica particles was evaluated using two different concentrations of DOX (20 and 40 mg/ml) and B2 silica particle group. As seen in FIG. 2A, the absorbance spectrum of the particles showed a peak at the same wavelength as the positive control (doxycycline alone), while particles alone did not display any peak. Consistent with expectations, the encapsulation efficiency analysis revealed that the encapsulation at 40 mg/ml had a higher efficiency than 20 mg/ml (p<0.05) and, for this reason, it was decided to use a DOX at 40 mg/ml in all the following analyses. During this assay, it was also analysed if the pH influenced the encapsulation, considering the negative charge of silica at neutral pH; however, the encapsulation efficiency could not be measured due to the tendency of DOX to precipitate in neutral or alkaline solutions.

Subsequently, the encapsulation efficiency of the silica particles was evaluated according to the different synthesis parameters (B2, B5 and B7). As a result, there is no statically significance between the different groups (p>0.05), displaying relatively low encapsulation capacity (FIG. 2B). Based on these findings, it was decided to proceed with B2 particles, henceforth referred to as silica submicron particles (SiSMP). To further enhance the encapsulation efficacy, it was decided to compare and evaluate the particles in dry and in aqueous solution (FIG. 2C), since all previous analyses were performed with particles suspension. As a result, the encapsulation capacity was greater when silica particles were pre-dried (p<0.05) and, therefore, silica particles were dried prior to encapsulation throughout the present study.

Even dried SiSMP showed significant loading capacities, two different surfactants were incorporated during synthesis to control pore sizes, as above explained. This was performed to increase the encapsulation capacity of silica particles. FIG. 2D shows the encapsulation efficiency of the synthesized silica particles with and without surfactants. It can be observed the significant higher drug encapsulation capacity of those particles synthesized with surfactants, loading 314.1 ± 25.3 µg and 272.3 ± 42.1 µg of DOX for SiSMP+SDS and SiSMP+CTAB, respectively. In contract, unmodified SiSMP only loaded 59.1 ± 8.4 µg of DOX.

This higher encapsulation capacity was correlated to the fact that SDS and CTAB are agents that induce the formation of pores in the particles, thus increasing pore size and surface area inside them. In addition, there are no statistically significant when comparing particles synthesized with SDS or CTAB. As mentioned before, this should be further studied as each surfactant induces different particle morphologies and pore structures, SDS inducing particles with a star-shaped structure, while CTAB inducing hexagonal pores that cross the entire particles. Based on these results, SiSMP, SiSMP+SDS and SiSMP+CTAB particles were further analyzed.

### Example 4: Doxycycline released from SiSMP

To study the doxycycline release 50 µg of SiSMP were suspended in 4000 µL of PBS solution into a falcon tube. Then, 1000 µL of the solutions were transferred in three different Eppendorf tubes. All the Eppendorf tubes were sealed with parafilm, covered with aluminium foil, and introduced in a grip bag at room temperature. To measure the released doxycycline, samples were assessed up to 2, 6, 24, and 48 hours. At each time point, release DOX was quantified as the previous section.

The in vitro release profiles of DOX from SiSMP, SiSMP+SDS and SiSMP+CTAB particles at a neutral pH is shown in FIG. 2E. For all formulations, a characteristic initial burst release was observed within the first two hours, releasing 99% of DOX. There was a significant difference on the DOX release amount of SiSMP+SDS and SiSMP+CTAB compared to SiSMP due to the initial DOX encapsulation (p<0.05). After 24 and 48 hours release, DOX release slightly declined for all conditions, possibly indicating drug reabsorption into the mesopores.

Previous studies also reported an initial burst release at the first hours from silica forms. This release profile can be due to the diffusion of drug molecules through the mesopores generated by surfactants or it could also be due to the diffusion of molecules trapped on the particle surface. Moreover, it was also observed that the drug release of the particles was pH-dependent, as pH decreases, doxycycline release increases.

### Example 5: Biomineralization capacity

The biomineralisation capacity of SiSMP, SiSMP+SDS and SiSMP+CTAB was evaluated after in a simulated body fluid (SBF) solution (FIG. 2F). After 21 days incubation, SiSMP and SiSMP+SDS particles did not demonstrated any change in their morphology or any presence of calcium precipitation. On the contrary, SiSMP+CTAB particles induced apatite-like crystals precipitates with spherical forms (FIG. 2F and G). Complementary, the presence of calcium and phosphate in the precipitates was confirmed by an energy dispersive X-ray (EDX) spectroscopy (FIG. 2H).

It should be noted that silica is considered a bioactive material, so it can induce the formation of calcium. However, silica particles obtained by the Ströber method did not display this feature. this biomineralisation capacity of SiSMP+CTAB may be attributed to a combination of factors (pore size, pore geometry and zeta potential) and synthesis parameters (acidic washes instead of surfactant calcination). There is a previous study that reported silica particles modified with CTAB with biomineralisation capacity; however, this was obtained by the insertion of amorphous calcium phosphate precursors (ACP).

### Example 6: In vitro antibacterial activity evaluation of the DOX loaded SiSMPs

### Minimal Inhibitory Concentration (MIC)

For the DOX MIC development, a 200 µg/ml DOX stock dilution was prepared to prepare serial dilutions: 200, 100, 50, 25, 12.5, 6.25, 3.12, 1.58, and 0 µg/ml. An overnight incubated inoculum of *E. faecalis* was prepared at an O.D of 0.2 ± 0.01. Then, 100 µL of bacteria and 100 µL of each concentration were added in a 96 well-plate and measured overnight every 15 minutes in the spectrophotometer at 600 nm.

The minimal inhibitory concentration (MIC) of doxycycline to *E. faecalis* was studied to determine the susceptibility of this specific strain to this antibiotic. As expected, the growth curve increased as the drug concentration decreased (FIG. 3A). In the graph, a black dashed line was used as a reference to facilitate the determination of the MIC. As a result, it was obtained that the minimum inhibitory concentration of doxycycline was found between 3.12 and 6.25 µg/mL, which was when the curve begins to grow. These results were consistent with a previous study which determined MIC at 4-6 µg/mL.

### Agar diffusion test

An inoculum of *E. Faecalis* was prepared at an optical density of 0.10 ± 0.01. Then, 100 µL of bacteria were seeded in different agar plates, and 50 µL of 5 mg/ml DOX-SiSMP of different conditions were placed in the middle of different agar plates. The assay was performed, using a positive control (commercially available calcium hydroxide, Calcicur) and a negative control (empty SiSMP), and then incubated at 37 °C under anaerobic conditions for 24h. Inhibition area was measured using Image J (NIH).

An agar diffusion test (ADT) of DOX+SiSMP, DOX+SiSMP+SDS, and DOX+SiSMP+CTAB was performed to qualitatively assess their antibacterial capacity (FIG. 3A). In addition, a negative control (SiSMP without doxycycline) and a commercially available intracanal medication based on calcium hydroxide (Calcicur) were used. This assay revealed that DOX+SiSMP, DOX+SiSMP+SDS, DOX+SiSMP+CTAB displayed an inhibition area, while SiSMP alone did not show any antibacterial activity: Mover, Calcicur did not show any effect against *E. faecalis* in ADT due to the buffering effect of agar. For a more quantitative analysis, the inhibition area of the three different particles was measured, observing that the DOX loaded particles, synthesized with either surfactant, had significantly greater antibacterial activity than those without (p<0.05, FIG. 3B). However, there was no statistically significant difference between DOX+SiSMP+SDS and DOX+SiSMP+CTAB (p>0.05). Considering the semi-quantitative nature of the agar diffusion method, it was deemed necessary to conduct further quantitative tests to ensure these results.

Similarly, an inoculum of *P. Gingivalis* was prepared at an optical density of 0.10 ± 0.01. Then, 100 µL of bacteria were seeded in different agar plates, and 50 µL of 5 mg/ml DOX-SiSMP of different conditions were placed in the middle of different agar plates. The assay was performed, using a positive control (commercially available calcium hydroxide, Calcicur) and a negative control (empty SiSMP), and then incubated at 37 °C under anaerobic conditions for 24h. Inhibition area was measured using Image J (NIH).

An agar diffusion test (ADT) of DOX+SiSMP, DOX+SiSMP+SDS, and DOX+SiSMP+CTAB was performed to qualitatively assess their antibacterial capacity (FIG. 6D). In addition, a negative control (SiSMP without doxycycline) and a commercially available intracanal medication based on calcium hydroxide (Calcicur) were used. This assay revealed that DOX+SiSMP, DOX+SiSMP+SDS, DOX+SiSMP+CTAB displayed an inhibition area. For a more quantitative analysis, the inhibition area of the three different particles was measured, observing that the DOX loaded particles, synthesized with either surfactant, had significantly greater antibacterial activity than those without (p<0.05, FIG. 6C). However, there was no statistically significant difference between DOX+SiSMP+SDS and DOX+SiSMP+CTAB (p>0.05).

### Assessment of the antibacterial activity in 2D in vitro culture

The antibacterial activity of the different DOX+SiSMP particles was evaluated in vitro against an *E. faecalis* biofilm and a *P. Gingivalis* biofilm formed on tissue culture plastic. All the studied groups revealed significant reduction of the metabolic activity compared to the control in a concentration and particle type manner (FIG. 3C and FIGs. 6A and 6B). As can be seen, the higher the concentration of particles used, the lower the metabolic activity of *E*. *faecalis* and *P*. *Gingivalis.* Therefore, the particle concentration that showed a lower metabolic activity was 5 mg/mL in the case of the three types of particles, obtaining a statistically significance difference compared to the positive control (p<0.05). In addition, Calcicur exhibited a moderate reduction of metabolic activity compared to control, similar tends were exhibited by 1.0 mg/ml DOX+SiSMP and 0.5 mg/ml DOX+SiSMP+SDS and DOX+SiSMP+CTAB. Comparing the modified particles, DOX+SiSMP+SDS and DOX+SiSMP+CTAB demonstrated higher reduction of the metabolic activity compared to the same concentration of DOX+SiSMP.

To quantify the colony formation units, the supernatants of the different concentrations (5 mg/ml) in the 96-well plate, mentioned above, were diluted in different Eppendorf tubes by the serial dilution method up to 10⁴. Lately, 10 µL of each Eppendorf tube was added in triplicates in different agar plates. A positive control (Calcicur) and a negative control (empty SiSMP) were also used.

To further validate the previous results, the antibacterial capacity of DOX loaded SiSMPs was confirmed by quantifying the colony-forming units (CFU) in an assay conducted at a concentration of 5 mg/mL, see FIG. 3C for *E. faecalis* and FIGs. 6A and 6B for *P. Gingivalis.* This concentration was selected based on prior tests indicating optimal results. All conditions showed a significant reduction in CFU compared to the control (p<0.05). Moreover, SiSMP+SDS and SiSMP+CTAB showed a greater reduction in CFU compared to SiSMP (p<0.05). This could be attributed to the higher DOX loading capacity, which is controlled by the particle pore size. No statistically significant difference was observed between SiSMP+SDS and SiSMP+CTAB (p>0.05).

### Example 7: Ex vivo infiltration capacity evaluation of the different SiSMPs

The infiltration capacity of the different SiSMPs through the dentinal canals was evaluated in an ex vivo model. Calcicur, SiSMP+SDS and SiSMP+CTAB were previously labelled with rhodamine. Then, particles were injected within the root canal and, subsequently, teeth were cut into thin laminas and examined with a confocal microscope to assess their infiltration into the dentinal canals.

For the model preparation, each tooth was instrumentalized to expose the root canal, scanned to obtain a digital file for designing a custom cutting guide. Then, all the cutting guides were 3D printed using Ultimaker S5 and tooth were divided in three blocks (coronal, medial and apical) using a mechanical precision saw and the cutting guides.

To evaluate the dentin tubules infiltration capacity of the different particles, it was decided to carry out a staining test with rhodamine. SiSMP+SDS, SiSMP+CTAB and control (Calcicur) were incubated in a 0.5 µg/ml rhodamine solution for 5 min and centrifuged at 4°C at 10000 rpm. The supernatant was discarded, and rhodamine-stained nanoparticles were washed once with PBS. Finally, stained nanoparticles at 5 mg/ml were transferred to different syringes. For this test, uncut teeth were used for each condition, injecting the rhodamine-stained particles within the root canal. After 24 hours, the teeth were cut using the cutting guides previously designed and 3D printed. Each tooth slide was analysed using a confocal microscope (DMi8, Leica), excitation wavelength at 546 nm and an emission wavelength at 567 nm.

To evaluate the dentin tubules infiltration capacity of the different particles, it was decided to carry out a staining test with rhodamine. SiSMP+SDS, SiSMP+CTAB and control (Calcicur) were incubated in a 0.5 µg/ml rhodamine solution for 5 min and centrifuged at 4°C at 10000 rpm. The supernatant was discarded, and rhodamine-stained nanoparticles were washed once with PBS. Finally, stained nanoparticles at 5 mg/ml were transferred to different syringes. For this test, uncut teeth were used for each condition, injecting the rhodamine-stained particles within the root canal. After 24 hours, the teeth were cut using the cutting guides previously designed and 3D printed. Each tooth slide was analyzed using a confocal microscope (DMi8, Leica), excitation wavelength at 546 nm and an emission wavelength at 567 nm.

Calcicur, SiSMP+SDS and SiSMP+CTAB infiltrated the dentin canals (FIG. 4A). However, Calcicur showed some areas without fluorescence, which indicates the absence of the material in these areas. On the other hand, SiSMP+SDS and SiSMP+CTAB showed a great infiltration capacity, penetrating more than 600 µm. However, differences were only statistically significant for SiSMP+CTAB (p<0.05) due to the large deviation for Calcicur and SiSMP+SDS. Some studies also reported a certain inability of infiltration by Calcicur compared to particles. This could be since the size of the particles was smaller, making it easier to penetrate the dentin canals.

### Example 8: Ex vivo antibacterial activity assessment of the different SiSMPs

Based on the antibacterial activity in 2D culture and the infiltration assay results, it was decided to assess the antibacterial capacity of the particles synthesized with surfactant (SDS and CTAB) and loaded with DOX in an ex vivo model. As the ex vivo model implies human teeth and a biofilm developed for 21 days that resembles clinical scenarios, it represents a more realistic and challenging scenario for the antibacterial treatment due to the anatomical structure.

Teeth were rehydrated in BHI media and incubated in agitation overnight at 37°C. Then, an inoculum of *Enterococcus faecalis* with an optical density of 0.10 ± 0.01 was prepared and 2 mL were added in each tooth and incubated at 37°C for 21 days. Replacing culture media every 48 hours. Then, 3 mL of DOX-SiSMP at 5 mg/ml were injected in the root canal and incubated at 37°C for 72h. To study the DOX-SiSMP antibacterial efficacy, all the samples were washed with phosphate buffered saline (PBS). Then, 1 mL of resazurin was added in each tube and incubated at 37°C for 15 min. Lately, 100 µL of each tube was transferred to a black 96-well plate to measure the fluorescence in the spectrophotometer at 560 nm excitation wavelength and at 590 nm emission wavelength.

To quantify the colony-forming units, the different tooth parts were added in 1 mL of PBS, vortexed for 5 min and ultrasonicated for 1 min. Then, the solutions were transferred into Eppendorf tubes and serial dilution up to 10⁴ were prepared. Then, 10 µL of each Eppendorf tube was added in triplicates in different agar plates. A negative control (bacteria alone) was also used.

To observe the bacteria viability, the LIVE/DEAD BacLight Bacterial Viability Kit was used. Then, 20 µL of SYTO9 to stain the live bacteria and 20 µL of Propidium Iodide to stain the dead ones, were added in a 15 mL falcon with 3 mL of PBS. Lately, the medium of all the tooth samples were discarded, and 1 mL of the solution was transferred in all the tubes containing only the apex part for an incubation at 37°C for 15 min. Finally, to characterize the different samples, apexes were placed in micro-dishes and observed at 10 and 63x under the DMI8 (Leica) confocal microscopy. Moreover, the LAS X Life Science software was used.

The metabolic activity of *E. faecalis* in the presence of Calcicur, SiSMP+SDS and SiSMP+CTAB was also evaluated. As shown in FIG. 4B, all three groups showed a reduction in metabolic activity compared to the positive control (bacteria alone) (p<0.05). In addition, the Calcicur induced a lower metabolic activity than the DOX loaded silica particles and SiSMP+CTAB obtained the lowest metabolic activity. Moreover, the quantification of CFU was also carried out in the ex vivo models (FIG. 4B), showing a significant reduction for SiSMP+SDS and SiSMP+CTAB (p<0.05). In addition, SiSMP+CTAB induced a greater CFU reduction compared to SiSMP+SDS (p<0.05). It is important to note that this CFU quantification assay did not turn out to be very precise for this ex vivo model, since many bacteria did not come off from the dentine channels; even sonication and vigorous vortex were employed.

Live/Dead assay was used to further confirm the ex vivo antibacterial activity of the different silica particles and Calcicur in the apical region of teeth (FIG. 4C). Control and Calcicur demonstrated mainly live bacteria (green staining) with some dead bacteria (red staining), only for Calcicur. In contrast, SiSMP+SDS and SiSMP+CTAB demonstrated most bacteria stained in red and some green, confirming the higher antimicrobial effects of DOX loaded SiSMP+SDS and SiSMP+CTAB compared to Calcicur, a commercially available intracanal medication based on calcium hydroxide. It is worth pointing out that both SiSMP+SDS and SiSMP+CTAB exhibited red stained bacteria within the dentinal channels, confirming the hypothesis that SiSMPs can infiltrate within the dentinal channels and release doxycycline to eliminate bacteria. The infiltration capacity of endodontic treatments has been reported to be essential to guarantee a more effective bacterial elimination in complex anatomical areas [36].

### Example 9: Toxicity evaluation of the different SiSMPs

Human foreskin fibroblasts (HFF-1) were purchased from American Type Culture Collection (ATCC). Cells were cultured with Dulbecco's Modified Eagle's Medium (DMEM) supplemented with 15% of Fetal Bovine Serum (FBS) and 1% of penicillin-streptomycin at 37°C. Then, 5 [X 10] ^3 cells/well were seeded in a 96-well plate an incubated for 24 hours. Then, cells were exposed to cell media containing different silica particles with concentrations (of 1, 5, 10, 50, 100, 200, 300 µg/mL) for 1, 3 and 7 days. A control (0 µg/mL of silica particles) was used in each experiment. To evaluate cell viability, a metabolic activity assay was performed. At each time point, the medium was gently removed, 100 µL of resazurin was added in each well and incubated at 37 °C. Lately, 100 µL of each tube was transferred to a black 96-well plate to measure the fluorescence in the spectrophotometer at 560 nm excitation wavelength and at 590 nm emission wavelength.

For statistical analysis, numerical data are expressed as mean ± standard deviation. All the statistical analysis was performed using GraphPad Prism 9. A one-way ANOVA was used for greater than two samples and a t test for two samples, after confirming the normal distribution of each sample population (Shapiro-Wilk test of normality). In addition, Dunnett's post-hoc was used for multiple comparisons. For the analysis of fibroblast metabolic activity, Kruskal-Wallis test was used. Statistical significance was accepted at p< 0.05.

The effect of three distinct silica particle types (SiSMP, SiSMP+CTAB, and SiSMP+SDS) on human fibroblasts was evaluated for 1, 3 and 7 days. FIG. 5 shows data for day.

All conditions preserve the characteristic spindle-like morphology of fibroblasts (FIG. 5A), indicating the absence of cellular stress or toxicity effects across all particle conditions for the different silica particles (1, 5, 10, 50, 100, 200 and 300 µg/mL). Moreover, at higher particle concentrations, some small black dots can be observed suggesting silica particle precipitation, being more evident for day 1 and 3.

Furthermore, FIG. 5B illustrates the metabolic activity of cells. The graph demonstrates a dose-dependent cytotoxic effect, evidenced by a statistically significant decrease in fibroblasts metabolic activity at the highest particle concentration (300 µg/mL) compared to the control. This dose-dependent decline in metabolic activity could suggest the direct relationship between silica particle concentration and cytotoxicity. However, no significant deviations were observed when comparing other particle concentrations or the different types of silica particles. Moreover, metabolic activity of fibroblast increased over time with limited increase or even some reduction at day 3.

## Claims

1. A substance for use in the treatment of an endodontic infection, wherein the substance comprises submicron silica particles.

2. The substance according to claim 1, wherein the submicron silica particles are spherical and/or polygonal and/or have a distribution size between 400 and 1200 nm.

3. The substance according to claims 1 or 2, wherein the submicron silica particles incorporate a cationic surfactant, an anionic surfactant and/or combinations thereof.

4. The substance according to claim 3, wherein the cationic surfactant is cetyl trimethyl ammonium bromide and/or the anionic surfactant is sodium dodecyl sulfate.

5. The substance according to claim 4, wherein the cationic surfactant is cetyl trimethyl ammonium bromide.

6. The substance according to claim 5 for use in the regeneration of dental bone tissue.

7. The substance according to claim 6, wherein the regeneration of dental bone tissue involves the formation of calcium apatite.

8. The substance according to any one of the previous claims, wherein the submicron silica particles encapsulate an antibiotic.

9. The substance according to claim 8, wherein the antibiotic is doxycycline.

10. The substance according to any one of the previous claims, wherein the endodontic infection is caused by *Enterococcus faecalis, Porphyromonas Gingivalis, Fusobacterium Nucleatum, Pseudomonas aeruginosa,* other *Streptococci* and/or *Prevotella* species or combinations thereof.

11. The substance according to any one of the previous claims, wherein the endodontic infection is a secondary or persistent endodontic infection.

12. The substance according to any one of the previous claims, wherein the endodontic infection is located in the dentinal tubule.

13. A method to obtain the substance as defined in any one of claims 1 to 12, comprising the following steps:
a) Mixing an alcohol with ammonium hydroxide,
b) Adding a silicon alkoxide to the mixture,
c) Stirring the resulting mixture to obtain a solid and a liquid phase,
d) Separating the solid phase by centrifugation and removing of the resulting supernatant,
e) Redispersing the solid phase in water, and
f) sonicating the re-dispersed solid phase.

14. The method according to claim 13, wherein the alcohol is pure ethanol and/or the silicon alkoxide is tetraethyl orthosilicate.

15. The method according to claims 13 or 14, further comprising a step of adding a solution in alcohol of a surfactant prior to step c) and a step of adding hydrochloric acid after step f).
